(19) Europäisches Patentamt
European Patent Office
Office européen des brevets

(11) Veröffentlichungsnummer: **0 240 907 B1**

(12) # EUROPÄISCHE PATENTSCHRIFT

(45) Veröffentlichungstag der Patentschrift: **29.05.91**

(51) Int. Cl.$^5$: **A61K 31/135**

(21) Anmeldenummer: **87104733.8**

(22) Anmeldetag: **31.03.87**

(54) **Verwendung von trans-4-[(2-Amino-3,5-dibrom-benzyl)-amino]cyclohexanol.**

(30) Priorität: **02.04.86 DE 3610997**

(43) Veröffentlichungstag der Anmeldung:
**14.10.87 Patentblatt 87/42**

(45) Bekanntmachung des Hinweises auf die Patenterteilung:
**29.05.91 Patentblatt 91/22**

(84) Benannte Vertragsstaaten:
**AT CH DE IT LI**

(56) Entgegenhaltungen:
**EP-A- 0 125 634**

**ARZNEIM. -FORSCH./DRUG RES., Band 28, Nr. 5a, 1978, Seiten 931-933; H. HOFFMANN: "Inhalationstherapie mit dem neuen Sekretolytikum Ambroxol (Metabolit VIII des Bromhexin) in der Hals-Nasen-Ohren-Praxis"**

**VALSALVA, Band 60, 1984, Seiten 161-168; E. TINELLI et al.: "L'ambroxol nei processi infiammatori dei seni paranasali"**

**THERAPIEWOCHE, Band 35, 1985, Seiten 5316-5321, G. Braun Verlag Zeitschriften; H.-D. RENOVANZ: "Die Pharmakotherapie entzündlicher Rhinopathien"**

**Rote Liste, 1985, 71100**

(73) Patentinhaber: **KREWEL-WERKE GMBH**
**Krewelstrasse**
**W-5208 Eitorf(DE)**

(72) Erfinder: **Schierstedt, Detlef, Dr.**
**Henri-Dunant-Strasse 2**
**W-5205 St.Augustin 3 Meindorf(DE)**

(74) Vertreter: **Strehl, Schübel-Hopf, Groening**
**Maximilianstrasse 54 Postfach 22 14 55**
**W-8000 München 22(DE)**

EP 0 240 907 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von trans-4-[(2-Amino-3,5-dibrom-benzyl)-amino]cyclohexanol zur Herstellung eines Arzneimittels, welches neue therapeutische Möglichkeiten in der Rhinologie eröffnet.

Es ist bisher üblich, zur Behandlung des vulgären Schnupfens (Rhinitis catarrhalis) Sympathomimetica lokal in Form von Nasentropfen oder Nasensprays anzuwenden, um eine Schleimhautabschwellung zu erreichen. Sympathomimetica besitzen jedoch zahlreiche unerwünschte Nebenwirkungen und können infolge einer Resorption zu Herzklopfen und Atemstörungen führen. Darüber hinaus verursachen sie die Austrockung der Nasenschleimhaut und bei längerer Anwendung kommt es zu einer dauerhaften Nasenschleimhaut-Epithel-Schädigung, die als Rhinopathia medicamentosa bekannt ist (Apotheker-Journal 12, 30-34 (1985) Otto Hoffmanns Verlag, München).

Der Erfindung liegt demgegenüber die Aufgabe zugrunde, ein Mittel zur lokalen Behandlung von Rhinitiden, insbesondere Rhinitis catarrhalis, zur Verfügung zu stellen, welches nicht mit den für Sympathomimetica bekannten Nebenwirkungen behaftet ist und welches keine Schädigung der Nasenschleimhaut verursacht.

Als pharmazeutische Wirksubstanz für eine andere Indikation war trans-4-[(2-Amino-3,5-dibrombenzyl)-amino]cyclohexanol (Trivialbezeichnung Ambroxol) bereits wegen seiner Wirkung zur Stimulierung des oberflächenaktiven Systems der Lunge und der tracheobronchialen Sekretproduktion zur Behandlung von Erkrankungen des respiratorischen Systems eingesetzt worden. So wurde Ambroxol zur Behandlung von akuten und chronischen Atemwegserkrankungen, insbesondere akuten Schüben chronischer Bronchitiden, Asthma bronchiale, Bronchiektasien, Laryngitis, Sinusitis und Rhinitis sicca; prä- und postoperativ, vor allem in der Alterschirurgie sowie speziell in der Intensivmedizin zur Vermeidung pulmonaler Komplikationen angewendet.

Zu diesem Zweck wurde Ambroxol oral, parenteral oder in Inhalatform verabreicht.

So wird beispielsweise in der DE-A-34 25 007 ein Addukt aus Ambroxol und Theophyllin-7-acetat genannt, welches in Form von Kapseln, Dragees, Sirup oder Injektionslösungen oder in Form von Aerosol zur Anwendung kommen soll, um Erkrankungen des Atmungstrakts, wie akute und chronische Bronchitits, Bronchialasthma und Lungenemphysem zu behandeln. Gemäß DE-A-33 17 530 werden als Antischnarchmittel Sekretolytika eingesetzt, die sonst als Hustenmittel und Bronchialmittel bekannt sind. Auch bei dieser Anwendungsart wird die an sich bekannte sekretolytische Wirkung von Ambroxol und anderen Mitteln ausgenutzt.

Bei Anwendung von Ambroxol muß die Dosis 50 bis 100 mg betragen. Berücksichtigt man, daß Ambroxol nur in 1 %iger Lösung stabil ist und alle konzentrierteren Lösungen bei tiefen Temperaturen zur Abscheidung neigen, so zeigt dies, daß das Antischnarchmittel in der Praxis in einer Dosis von 5 bis 10 ml zur Anwendung kommen müßte, die völlig unrealistisch ist.

Die Tatsache, daß man bisher Ambroxol ausschließlich als Bronchialmittel angewendet hat, geht auch aus der Veröffentlichung in "Arzneimittelforschung/Drug Research", 28 (I), (1978), Seiten 931 bis 933, hervor. Gemäß dieser Literaturstelle wird Ambroxol in 1 %iger Emser Sole als Inhalat eingesetzt. Die klinischen Befunde bestätigen, daß Ambroxol wirksam bei Patienten mit tracheobronchialem oder sinubronchialem Syndrom war, daß es aber im Fall von Patienten, die zusätzlich Rhinitis hatten, notwendig war, zusätzlich zur Behandlung der Rhinitis Vasokonstriktoren- und Antihistaminika- haltige Schnupfenmittel zu verabreichen, um die Behandlung durchführen zu können.

Der vorstehend beschriebene Stand der Technik zeigt, daß Ambroxol bisher ausschließlich in Form von oral zu verabreichenden Mitteln, Injektionslösungen oder Inhalaten zur Behandlung von Krankheiten des Bronchialsystems eingesetzt wurde. Wenn erkannt worden wäre, daß die Verbindung - bei ihrer unmittelbaren Anwendung auf die Nasenschleimhaut - wirksam ist, wäre die gleichzeitige Verabreichung Schnupfenmitteln nicht erforderlich geworden.

Bei der bisherigen Anwendung als Inhalat wird die Arzneistofflösung so fein vernebelt (die Tröpfchengröße liegt unter 10 $\mu$m), daß sie hauptsächlich in die Bronchien gelangt. Partikel dieser Größenordnung passieren den Nasenraum nahezu ungehindert, so daß dort keine therapeutisch wirksamen Ambroxol-Konzentrationen entstehen können. Wird dagegen ein Arzneimittel in Form eines Nasensprays versprüht, ist die Tropfengröße etwa 1 bis 2 Zehnerpotenzen größer als bei einem Inhalat, so daß der größte Teil des Wirkstoffes im Nasenraum verbleibt und dort seine volle Wirkung entfalten kann.

Angesicht des vorstehend erläuterten Standes der Technik war es daher überraschend, daß erfindungsgemäß festgestellt werden konnte, daß die an sich bekannte Verbindung Ambroxol bei lokaler Anwendung auf die Nasenschleimhaut bei Rhinitiden einen ausgezeichneten therapeutischen Effekt zeigt, ohne daß es mit den unerwünschten Nebenwirkungen der für diesen Anwendungszweck bekannten Sympathomimetica behaftet ist.

Gegenstand der Erfindung ist somit die Verwendung von trans-4-[(2-Amino-3,5-dibrom-benzyl)-amino]-cyclohexanol der Formel I

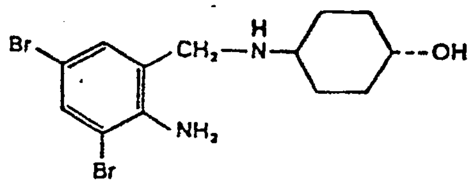

(I)

oder eines pharmazeutisch geeigneten Salzes dieser Verbindung zur Herstellung eines die Nasenschleimhaut abschwwellenden Arzneimittels zur lokalen Behandlung von Rhinitiden in Form von Nasenspray oder Nasentropfen.

Die erfindungsgemäß zur Behandlung von Rhinitiden verwendete Wirksubstanz der oben angegebenen Formel I ist eine unter der Bezeichnung Ambroxol bekannte Verbindung. Sie hat folgende Merkmale:
Molekulargewicht: 414,58
Die Substanz ist farb- und geruchlos, sie hat einen leicht bitteren Geschmack.

Die Verbindung wird meist in Form von pharmazeutisch geeigneten, insbesondere wasserlöslichen Salzen eingesetzt. Besonders bevorzugt wird das Hydrochlorid.

Es ist überraschend, daß Ambroxol, wenn es in Form von Nasentropfen oder eines Nasensprays lokal appliziert wird, ein hervorragendes Therapeutikum für die Behandlung verschiedenartigster Rhinitiden darstellt. So ist das erfindungsgemäße Mittel äußerst wirksam bei der Behandlung des vulgären Schnupfens (Rhinitis catharralis).

Die lokale Verträglichkeit von Ambroxol ist sehr gut, da beim Kaninchen-Augen-Test mit einer 1%-igen Ambroxol-HCL-Lösung keine Reizung des Kaninchenauges festgestellt werden konnte (1).

Das erfindungsgemäß hergestellte Mittel in Form von Nasensprays oder Nasentropfen ist nicht nur außerordentlich gut wirksam, sondern auch frei von den schwerwiegenden Nebenwirkungen der bisher bekannten Mittel auf Basis von Sympathomimetica. In letzter Zeit werden die Nebenwirkungen von Schnupfenmitteln auf Basis von Sympathomimetica immer stärker beachtet und es ist bekannt, daß diese selbst bei Erwachsenen eine Schädigung der Nasenschleimhaut hervorrufen und zu Abgeschlagenheit und Müdigkeit führen.

Besonders schwerwiegend sind diese Nebenwirkungen bei Kleinkindern und Säuglingen, da bei der Anwendung solcher Nasentropfen die überschüssige Menge leicht in den Epi-, Meso-, Hypopharynz oder Larynx gelangt und eine echte Intoxikation hervorruft.

Das erfindungsgemäße Mittel ist frei von diesen Nebenwirkungen und eignet sich daher besonders gut für den Einsatz in der Kinderheilkunde.

Die erfindungsgemäß verwendete Verbindung der Formel I oder deren pharmazeutisch geeignete, insbesondere wasserlösliche Salze werden wirksam in einer Konzentration von 0,1 Gew.-% bis zur Löslichkeitsgrenze in einem wäßrigen Medium, insbesondere in Form 0,1 bis 1,5%-igen Lösungen, mehr insbesondere 0,325 bis 1,0%-igen Lösungen (in Gew.-%) in einer nasenschleimhautverträglichen galenischen Zubereitung eingesetzt. Die verabreichte Menge kann zwischen 0,02 bis 0,4 ml pro Sprühstoß betragen (vorzugsweise 0,05 bis 0,1 ml pro Sprühstoß).

Die galenische Zubereitung des erfindungsgemäß hergestellten Mittels kann nach den galenischen Methoden und Regeln erfolgen, wie sie für die Herstellung von wäßrigen Nasentropfen allgemein üblich sind (Sucker, H., P. Fuchs und P. Speiser: Pharmazeutische Technologie, Georg-Thieme-Verlag, Stuttgart (1978))

Dem Mittel können weitere Wirkstoffe zugesetzt werden, insbesondere Antibiotika, Corticoide, Sympathomimetica, Parasympatholytica, Antiphlogistica, Chemotherapeutica. Darüber hinaus kann das Mittel übliche Zusätze, wie Konservierungsmittel, enthalten, für die nachstehend Beispiele gegeben werden.

Tonie

a) Isotonie - leichte Hypertonie (insbesondere bevorzugt 400 Milliosmol)
Hilfsstoffe: z.B. Glykose, Sorbit, Mannit, Xylit, Natriumchlorid u.a.

Konservierung

b) mit Bezalkoniumchlorid, Thiomersal, Phenylquecksilbersalzen, Chlorhexidinacetat bzw. Glukonat, Chlorbutanol, PHB-Ester (besonders bevorzugt: Benzalkoniumchlorid) u.a.

pH-Wert

Einstellung auf einen physiologisch verträglichen pH-Wert möglichst nicht unter pH 5,5, vorzugsweise nicht unter pH 6,5 z.B. durch Natriumhydroxid, Natriumphosphat oder Natriumcitrat oder physiologisch verträgliche Puffersysteme, z.B. Phosphat oder Citratpuffer. Der obere Grenzwert entspricht der für Nasenspray üblichen pH-Grenze, der durch die Schleimhautverträglichkeit bestimmt ist.
Für die therapiegerechte Applikation ist der Einsatz eines Dosiersprühkopfes besonders bevorzugt.

Herstellungsbeispiele 1 bis 8

| Beispiele: | 1 | 2 | 3 | 4 | 5 | 6 | 7 | 8 |
|---|---|---|---|---|---|---|---|---|
| | g | g | g | g | g | g | g | g |
| Ambroxol HCl | 0,750 | 0,750 | 0,750 | 0,750 | 1,500 | 0,375 | 0,375 | 0,150 |
| Sorbit | 5,000 | | | 2,600 | 4,600 | 2,900 | | 5,500 |
| Glukose | | 5,000 | | | | | 2,900 | |
| Benzalkoniumchlorid | 0,014 | 0,014 | 0,014 | 0,014 | 0,014 | 0,014 | 0,014 | 0,014 |
| Natriumchlorid | | | 1,000 | | | | | |
| Tri-Natriumcitrat x5,5$H_2O$ | 0,053 | 0,053 | 0,063 | | 0,158 | | | 0,013 |
| Di-Natriumhydrogenphosphat 12$H_2O$ | | | | 2,500 | | 2,500 | 2,500 | |
| Citronensäure | | | | 0,290 | | 0,290 | 0,290 | |
| $H_2O$ ad | 100ml | 100ml | 100ml | 100ml | 100ml | 100ml | 100ml | 100ml |

Herstellung:

Nach dem Auflösen der Rezepturbestandteile in $H_2O$ wird die Lösung einer Sterilfiltration unterzogen und in die Spray-bzw. Nasentropfenflaschen abgefüllt und verschlossen.

Anwendungsbeispiel

Das wie vorstehend gemuaß Beispiel 4 zubereitete Ambroxol Nasenspray wurde an 15 Probanden mit der Diagnose Katarrh der Nasenschleimhaut, sowie an 3 Probanden mit Rhinitis sicca auf Wirksamkeit im Kurzversuch (1 - 3 Tage Therapiezeit) erprobt.

Ergebnis:

Der einfache Katarrh der Nasenschleimhaut konnte schon bei einmaliger Anwendung des Nasensprays sehr gut beeinflußt werden, so daß sich sofort nach Behandlungsbeginn die Sekretion der Nasenschleimhaut in den physiologischen Bereich bringen ließ und die bekannten anderen Symptome abnahmen.

Übereinstimmend gaben alle Probanden an, daß durch die 3mal tägliche Anwendung von Ambroxol Nasenspray das unangenehme "Nasenlaufen" völlig verschwunden war und Schleimabsonderung aus der Nase nur noch in einem normal üblichen Rahmen auftrat. Auch die bei Rhinopathien auftretende verminderte Nasenatmung konnte vollständig normalisiert werden.

Alle Patienten konnten die Therapie spätestens am dritten Tage nach Therapiebeginn beenden, ohne über weitere Befindlichkeitsstörungen im Nasenbereich klagen zu müssen.

Die 3 Probanden, die sich einer Ambroxol Nasenspray Kurztherapie bei Rhinitis sicca unterzogen, gaben übereinstimmend an, daß bei regelmäßiger Anwendung des Sprays das unangenehme Gefühl, welches bei der Rhinitis sicca im Nasenschleimhautbereich beschrieben wird, zum Teil schon nach 2 -3maliger Anwendung völlig verschwunden ist, auch während der Nacht (d.h. im therapiefreien Intervall).

Zusätzlich sei darauf hingewiesen, daß eine Ambroxol-Therapie der Nasenschleimhaut die gefürchtete Rhinopatia medicamentosa verhindert.

## Ansprüche

1. Verwendung von trans-4-[(2-Amino-3,5-dibrom-benzyl)-amino]cyclohexanol der Formel I

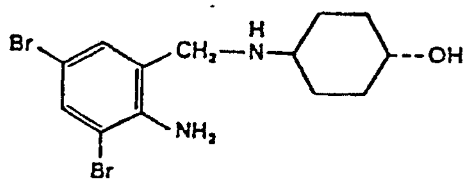

(I)

oder eines pharmazeutisch geeigneten Salzes dieser Verbindung zur Herstellung eines die Nasenschleimhaut abschwellenden Arzneimittels zur lokalen Behandlung von Rhinitiden in Form von Nasenspray oder Nasentropfen.

2. Verwendung nach Anspruch 1, dadurch gekennzeichnet, daß das herzustellende Mittel die Verbindung I oder ein pharmazeutisch geeignetes wasserlösliches Salz dieser Verbindung in einer Konzentration von 0,1 Gew.-% bis zur Löslichkeitsgrenze in einem wäßrigen Medium enthält.

3. Verwendung nach Anspruch 2, dadurch gekennzeichnet, daß die Konzentration der Verbindung I oder eines pharmazeutisch geeigneten Salzes dieser Verbindung auf 0,1 bis 1,5 Gew.-%, insbesondere 0,325 bis 1,0 Gew.-%, eingestellt wird.

4. Verwendung nach einem der Ansprüche 1 bis 3, dadurch gekennzeichnet, daß dem Mittel eine weitere Wirksubstanz zugesetzt wird, insbesondere eine Wirksubstanz, die aus der Gruppe der Antibiotika, Corticoide, Sympathomimetica, Parasympatholytica, Antiphlogistica und Chemotherapeutica ausgewählt ist.

5. Verwendung nach einem der Ansprüche 1 bis 4 zur Herstellung eines Mittels, welches die Verbindung der Formel I oder ein pharmazeutisch geeignetes Salz dieser Verbindung in isotonischer bis leicht hypertonischer Form, vorzugsweise von 400 Milliosmol, enthält.

6. Verwendung nach einem der Ansprüche 1 bis 5, dadurch gekennzeichnet, daß der pH-Wert des Mittels auf nicht weniger als 5,5, vorzugsweise nicht weniger als 6,5, eingestellt wird.

## Claims

1. Use of trans-4-[(2-amino-3,5-dibromobenzyl)-amino]cyclohexanol of the formula I

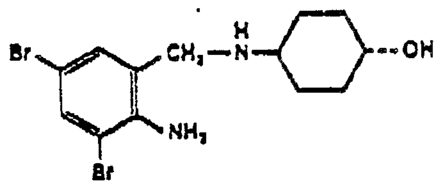

(I)

or a pharmaceutically suitable salt of this compound for the preparation of a medicament which detumesces the nasal mucosa for the local treatment of rhinitis, in the form of a nasal spray or nasal drops.

2. Use according to Claim 1, characterised in that the composition to be prepared contains the compound I or a pharmaceutically suitable water-soluble salt of this compound in a concentration of 0.1% by weight up to the solubility limit, in an aqueous medium.

3. Use according to Claim 2, characterised in that the concentration of the compound I or of a pharmaceutically suitable salt of this compound is adjusted to 0.1 to 1.5% by weight, in particular 0.325 to 1.0% by weight.

4. Use according to one of Claims 1 to 3, characterised in that a further active substance is added to the composition, in particular an active substance which is selected from the group comprising the antibiotics, corticoids, sympathomimetics, parasympatholytics, anti-inflammatories and chemotherapeutics.

5. Use according to one of Claims 1 to 4 for the preparation of a composition which contains the compound of the formula I or a pharmaceutically suitable salt of this compound in isotonic to slightly hypertonic form, preferably of 400 milliosmol.

6. Use according to one of Claims 1 to 5, characterised in that the pH of the composition is adjusted to not less than 5.5, preferably to not less than 6.5.

## Revendications

1. Utilisation du trans-4-[(2-amino-3,5-dibromobenzyl)amino]cyclohexanol de la formule I

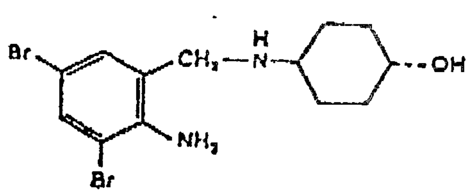

(I)

ou d'un sel pharmaceutiquement approprié de ce composé pour la préparation d'un médicament servant à désenfler les muqueuses nasales pour le traitement local de rhinites sous forme de spray nasal ou de gouttes nasales.

2. Utilisation selon la revendication 1, caractérisée en ce que le produit à préparer contient le composé I ou un sel hydrosoluble pharmaceutiquement approprié de ce composé dans une concentration de 0,1 % en poids jusqu'à la limite de solubilité dans un agent aqueux.

3. Utilisation selon la revendication 2, caractérisée en ce que la concentration du composé I ou d'un sel

pharmaceutiquement approprié de ce composé est ajustée à 0,1 jusqu'à 1,5 % en poids, notamment de 0,325 jusqu'à 1,0 % en poids.

4. Utilisation selon l'une des revendications 1 à 3, caractérisée en ce qu'une substance active supplémentaire est ajoutée au produit, notamment une substance active choisie parmi le groupe des antibiotiques, corticoïdes, sympathomimétiques, parasympatholytiques, anti-inflammatoires et substances chimiothérapeutiques.

5. Utilisation selon l'une des revendications 1 à 4 pour la préparation d'un produit qui contient le composé de la formule I ou un sel pharmaceutiquement approprié de ce composé sous la forme isotonique jusqu'à la forme légèrement hypertonique, de préférence de 400 milliosmols.

6. Utilisation selon l'une des revendications 1 à 5, caractérisée en ce que la valeur du pH du produit est ajustée à une valeur non inférieure à 5,5, de préférence non inférieure à 6,5.